# EUROPÄISCHE PATENTANMELDUNG

(11) **EP 2 666 868 A1**
(43) Veröffentlichungstag der Anmeldung: **27.11.2013**
(21) Anmeldenummer: 12004073.8
(22) Anmeldetag: 25.05.2012
(51) Int. Cl.: C12P 5/02, C12M 1/107

(54) **Verfahren und Vorrichtung zur Vergärung von stickstoffhaltiger Biomasse, insbesondere Hühnertrockenkot in einer Biogasanlage**

(71) Anmelder: Niederbacher, Michael, 39031 Bruneck (IT)
(72) Erfinder: Niederbacher, Michael, 39031 Bruneck (IT)
(74) Vertreter: Liebl, Thomas

(57) **Zusammenfassung**

Die Erfindung betrifft ein Verfahren und eine Vorrichtung zur Vergärung von stickstoffhaltiger Biomasse, insbesondere von Hühnertrockenkot, in einer Biogasanlage, bei dem die zu vergärende stickstoffhaltige Biomasse (1) in einem ersten Schritt einer Mischeinrichtung (10) zugeführt wird, in der diese mit einer definierten Menge eines Rezirkulates(31) vermischt wird, insbesondere dergestalt, dass die Stickstoffkonzentration des so erzeugten Biomasse/Rezirkulat-Gemisches (14) unter einem definierten Stickstoff-Schwellwert liegt, wobei das Biomasse/Rezirkulat-Gemisch (14) anschließend wenigstens einem Fermenter (15, 18) der Biogasanlage zugeführt wird, in dem Biogas erzeugt wird. In einem weiteren Verfahrensschritt wird Gärrest (21) aus einem Fermenter (15, 18) abgezogen und einer Abscheider- und/oder Separatoreinrichtung (22) zugeführt, in der der Gärrest in eine Gärrest-Feststoffphase (23) und in eine Gärrest-Flüssigphase (24) separiert wird, wobei lediglich die Gärrest-Flüssigphase (24) einer Stickstoff-Ausbringeinrichtung (26) zugeführt wird, in der der in der Gärrest-Flüssigphase (24) gelöste und/oder gebundene Stickstoff reduziert oder im Wesentlichen entfernt wird und eine stickstoffreduzierte Gärrest-Flüssigphase (29) erzeugt wird. Die stickstoffreduzierte Gärrest-Flüssigphase (29) wird anschließend der Mischeinrichtung (10) als Rezirkulat (31) zugeführt.

## Beschreibung

Die Erfindung betrifft ein Verfahren zur Vergärung bzw. Fermentation von stickstoffhaltiger Biomasse, insbesondere von Hühnertrockenkot in einer Biogasanlage nach dem Oberbegriff des Anspruchs 1. Weiter betrifft die Erfindung eine Vorrichtung zur Vergärung bzw. Fermentation von stickstoffhaltiger Biomasse, insbesondere von Hühnertrockenkot, in einer Biogasanlage nach dem Oberbegriff des Anspruchs 10.

Bei der Vergärung von stickstoffhaltiger Biomasse, wie beispielsweise von Hühnertrockenkot mit einem Stickstoffanteil von bis zu 30kg/t Frischmasse, besteht die Gefahr, dass sich der Stickstoff im Laufe des Abbaus der Biomasse in der Biogasanlage zum Beispiel in das, ab einer bestimmten Konzentration toxisch wirkende Ammonium umwandelt.

Um dem entgegenzuwirken, ist es bereits allgemein bekannt, stickstoffhaltige Biomasse, wie zum Beispiel Hühnertrockenkot zu Beginn des Fermentationsprozesses mit Frischwasser zu verdünnen bzw. anzumaischen, um hierdurch die Stickstoffkonzentration in der zu vergärenden Biomasse zu senken. Ein derartiger Einsatz von Frischwasser in einem Biogasanlagen-Fermentationsprozess ist jedoch regelmäßig unerwünscht, da er teuer und ökologisch uneffektiv ist.

Aus der EP 1929 024 B1 ist bereits ein Verfahren zur Herstellung von Biogas unter Verwendung eines Substrats mit hohem Feststoff- und Stickstoffanteil bekannt, bei dem die zu vergärende Biomasse zunächst in einem Zerkleinerer zerkleinert, angemaischt mit Bakterien geimpft und entstandenes Biogas abgezogen wird. Die zerkleinerte Biomasse wird anschließend in einem Mischer mit Rezirkulat angemaischt und somit das Substrat in einen pumpfähigen Zustand überführt. Anschließend wird diese pumpfähige Mischung in einem weiteren Inline-Zerkleinerer weiter homogenisiert, wobei in einem oder mehreren nachgeschalteten Abscheidern mit Rührwerk Hydrolyse und Säurebildung durch bakterielle Einwirkung ablaufen, anorganische Bestandteile separiert und verworfen werden und im Fermenter unter fortlaufender Durchmischung bei erhöhten Temperaturen Essigsäurebildung und Methanogenese ablaufen, um Biogas zu erzeugen. Das Biogas wird abgezogen und einer energetischen Nutzung zugeführt, während der entstandene Gärrest in zwei unbehandelte Teilströme aufgeteilt wird, von denen ein erster unbehandelter Teilstrom zum Anmaischen neuer Biomasse dient, während ein zweiter unbehandelter Teilstrom einer Substrataufbereitung unterzogen wird. Konkret wird während der Substrataufbereitung in einem Stripprozess Ammoniak bzw. Ammonium entzogen. Nach dem Stripprozess wird der Teilstrom als hygenisierter und stickstoffarmer Gärrest über einen Pufferbehälter kontinuierlich einem Separator zugeführt und in eine feste und flüssige Phase getrennt, wobei die flüssige Phase als Rezirkulat dem Mischer zugeführt wird, während die feste Phase entweder getrocknet oder als Suspension verwertet wird. Die Säurebildung und die Hydrolyse laufen dabei in einem oder mehreren nacheinander geschalteten Zyklonen mit einem Zentralrührwerk ab.

Gemäß einer weiteren alternativen Vefahrensführung ist in der EP 1 929 024 B1 vorgesehen, die in einem Zerkleinerer zerkleinerte, angemaischte, mit Bakterien geimpfte Biomasse mit Rezirkulat mit einem Mischer anzumaischen, um das Substrat in einen pumpfähigen Zustand zu überführen. Diese pumpfähige Mischung wird anschließend in einem sogenannten Inline-Zerkleinerer weiter homogenisiert. Weiter wird die pumpfähige Mischung in einer oder mehrerer nacheinander geschalteter Vorstufen mit Zentralrührwerk einer Hydrolyse und Säurebildung durch bakterielle Einwirkung unterzogen und die so entstandene Suspension einer Substrataufbereitung unterzogen, indem sie auf 70 bis 90 Grad Celsius aufgeheizt wird und ihr in einem Stripprozess Ammoniak/Ammonium entzogen wird. Der dem Stripprozess zu- und abgeführte Gärrest wird im Gegenstromverfahren abgekühlt bzw. vorgewärmt, wobei in einem dem Stripper nachgeschalteten Abscheider anorganische Bestandteile separiert verworfen werden, bevor das so erhaltene Substrat im Fermenter unter fortlaufender Durchmischung Biogas durch Fermentation erzeugt. Den Fermentern ist hier ein Separator nachgeschaltet, von dem ausgehend Flüssigphase zum Mischer zurückgeführt wird.

Bei einer derartigen Verfahrensführung ist jedoch ein erheblicher energetischer und auch apparatetechnischer Aufbau erforderlich, der über den gesamten Prozess eine sehr hohe Feststoffbelastung aufweist.

Demgegenüber ist es Aufgabe der vorliegenden Erfindung, ein Verfahren und eine Vorrichtung zur Verfügung zu stellen, mittels der bzw. mittels dem die Vergärung von stickstoffhaltiger Biomasse, insbesondere von Hühnertrockenkot, auf funktionssichere und apparatetechnisch einfache Weise durchgeführt werden kann.

Diese Aufgabe wird gelöst mit den Merkmalen der unabhängigen Patentansprüche. Vorteilhafte Ausgestaltungen hierzu sind jeweils Gegenstand der darauf rückbezogenen Unteransprüche.

Gemäß Patentanspruch 1 wird ein Verfahren zur Vergärung von stickstoffhaltiger Biomasse, insbesondere von Hühnertrockenkot, in einer Biogasanlage vorgeschlagen, bei dem die zu vergärende stickstoffhaltige Biomasse in einem ersten Verfahrensschritt einer Mischeinrichtung zugeführt wird, in der diese mit einer definierten Menge eines Rezirkulates vermischt wird, insbesondere dergestalt, dass die Stickstoffkonzentration des so erzeugten Biomasse/Rezirkulat-Gemisches unter einem definierten Stickstoff-Schwellwert liegt. Dieses Biomasse/Rezirkulat-Gemisch wird anschließend, vorzugsweise direkt und unmittelbar, das heißt ohne weitere Behandlung und/oder Aufbereitung, wenigstens einem Fermenter der Biogasanlage zugeführt, in dem Biogas erzeugt wird. In einem weiteren Verfahrensschritt wird Gärrest aus dem wenigstens einen Fermenter oder aus wenigstens einem der Fermenter abgezogen und vorzugsweise direkt und unmittelbar, das heißt ohne weitere Behandlung und/oder Aufbereitung einer Abscheider- und/oder Separatoreinrichtung zugeführt, in der der Gärrest in eine Gärrest-Feststoffphase und in eine Gärrest-Flüssigphase separiert wird, wobei lediglich die Gärrest-Flüssigphase einer Stickstoff-Ausbringeinrichtung zugeführt wird, in der der in der Gärrest-Flüssigphase gelöste und/oder gebundene Stickstoff, vorzugsweise in Form von Ammonium und/oder Ammoniak, reduziert oder im Wesentlichen entfernt wird und somit eine stickstoffreduzierte, vorzugsweise eine im Wesentlichen stickstofffreie, Gärrest-Flüssigphase erzeugt wird. Die so erhaltene stickstoffreduzierte bzw. stickstoffarme Gärrest-Flüssigphase wird anschließend zu definierten Zeiten und in definierter Menge der Mischeinrichtung als Rezirkulat zugeführt.

Mit einer derartigen erfindungsgemäßen Verfahrensführung wird die Vergärung von stickstoffhaltiger Biomasse, insbesondere von Hühnertrockenkot, ohne die Zugabe von Frischwasser (außer gegebenenfalls beim Anfahren der Biogasanlage) möglich, da im laufenden Biogasanlagenprozess durch die Rückführung des Rezirkulats sichergestellt werden kann, dass die Stickstoffkonzentration der zu vergärenden Biomasse unterhalb eines bestimmten Stickstoff-Schweilwertes gehalten werden kann. Besonders vorteilhaft bei der erfindungsgemäßen Verfahrensführung ist, dass hier der aus der Fermentationsstufe kommende Gärrest zuerst einer Abscheider- und/oder Separatoreinrichtung zugeführt wird, wodurch die Gärrest-Feststoffphase abgezogen wird und der Stickstoff-Ausbringeinrichtung somit lediglich die Gärrest-Flüssigphase zugeführt wird, sodass die Entfernung des gelösten bzw. gebundenen Stickstoffs dort wesentlich schneller und effektiver durchgeführt werden kann. Zudem kann diese Stickstoff-Ausbringeinrichtung aufgrund der fehlenden Feststoffphase entsprechend apparatetechnisch einfacher und kompakter und damit günstiger ausgebildet werden.

Zudem ist mit der erfindungsgemäßen Verfahrensführung sichergestellt, dass der Mischeinrichtung als Rezirkulat lediglich eine einfach pumpbare und damit einfach handhabbare stickstoffreduzierte bzw. stickstoffarme Flüssigphase zugeführt wird, wodurch die Anmaischung in der Mischeinrichtung im Hinblick auf das gezielte Absenken des Stickstoff-Schwellwertes funktionssicher durchgeführt werden kann.

Gemäß einer besonders bevorzugten Verfahrensführung ist vorgesehen, dass die Stickstoff-Ausbringeinrichtung durch eine wenigstens eine Strippereinrichtung gebildet ist, in der der gelöste und/oder gebunden Stickstoff der Gärrest-Flüssigphase in eine Gasphase überführt und wenigstens eine stickstoffreduzierte, insbesondere im Wesentlichen stickstoffreiche Gärrest-Flüssigphase erzeugt wird. Eine derartige Strippung kann auf einfache und funktionssichere Weise mit einer zuverlässigen Entfernung des Stickstoffs in Form einer Gasphase durchgeführt werden. Alternativ dazu kann die Stickstoff-Ausbringeinrichtung aber auch durch wenigstens eine Umkehrosmoseeinrichtung gebildet sein, in der der gelöste und/oder gebundene Stickstoff der Gärrest-Flüssigphase durch Umkehrosmose auf funktionssichere Weise abgetrennt und wenigstens eine stickstoffreduzierte, insbesondere im Wesentlichen stickstofffreie Gärrest-Flüssigphase erzeugt wird. Gegebenenfalls können auch mehrere derartiger Stickstoff-Ausbringeinrichtungen hintereinander geschaltet sein, wobei dann die einzelnen Stufen gleich oder aber auch unterschiedlich ausgebildet sein können.

Die stickstoffreduzierte Gärrest-Flüssigphase wird der Mischeinrichtung zum Beispiel je nach den vorherrschenden Betriebsbedingungen und zu vergärenden Biomassemengen zugeführt, was sowohl eine kontinuierliche Zuführung der Gärrest-Flüssigphase zur Mischeinrichtung als auch eine charchenweise Zuführung der stickstoffreduzierten Gärrest-Flüssigphase erforderlich machen kann. Um diesbezüglich eine einwandfreie Verfahrensführung sicherzustellen, wird gemäß einer weiter besonders bevorzugten Ausgestaltung vorgeschlagen, die stickstoffreduzierte Gärrest-Flüssigphase nach der Stickstoff-Ausbringeinrichtung in einem Zwischenspeicher oder Pufferbehälter zwischenzuspeichern, aus dem heraus zu definierten Zeiten eine definierte Menge der stickstoffreduzierten Gärrest-Flüssigphase abgezogen und der Mischeinrichtung als Rezirkulat zugeführt werden kann. Die Zuführung kann dabei gesteuert bzw. geregelt mittels einer entsprechenden Steuer- und/oder Regeleinrichtung, die den Gesamtprozess der Biogasanlage kontrollieren kann, erfolgen.

Gemäß einer weiteren besonders bevorzugten Ausgestaltung wird vorgeschlagen, dass die Gärrest-Feststoffphase von der Abscheider- und/oder Separatoreinrichtung abgezogen und einer Trocknereinrichtung zugeführt wird, in der die Gärrest-Feststoffphase auf einen definierten Trocksubstanzgehalt, insbesondere auf einen Trockensubstanzgehalt von 70 Prozent TS bis 95 Prozent TS, getrocknet wird. Mit einer derartigen Trocknung wird sichergestellt, dass auch der organisch gebundene Stickstoff aus dem Prozess ausgeschleust wird, wobei gemäß einer besonders bevorzugten Verfahrensführung vorgesehen ist, dass sowohl die Abluft aus der Trocknereinrichtung als auch der aus der Gärrest-Flüssigphase entfernte Stickstoff, der vorzugsweise in Form von Ammoniak und/oder Ammonium vorliegt, einem Gaswäscher zugeführt wird, in dem Ammoniumsulfat oder eine Ammoniumsulfatlösung und damit Dünger hergestellt wird. Die Herstellung von Ammoniumsulfat bzw. einer Ammoniumsulfatlösung kann dabei auf unterschiedliche Art und Weise erfolgen, zum Beispiel unter Anwesenheit von Schwefelsäure (H₂So₄) im Gaswäscher. Mit dem Gaswäscher können somit Staub, Geruch und Stickstoffemissionen minimiert werden und zudem als Endprodukt ein Dünger produziert werden.

Um insbesondere die Verweilzeit der zu vergärenden Biomasse in der Fermentationsstufe zu verringern, wird gemäß einer besonders bevorzugten weiteren Ausführungsvariante vorgeschlagen, die zu vergärende stickstoffhaltige Biomasse vor deren Zuführung zur Mischeinrichtung in einer Aufschluss- und/oder Zerkleinerungseinrichtung aufzuschließen bzw. zu zerkleinern und somit die Biomasse der Mischeinrichtung als aufgeschlossene und/oder zerkleinerte stickstoffhaltige Biomasse zuzuführen. Als Aufschluss- und/oder Zerkleinerungseinrichtung kann beispielsweise ein Extruder, zum Beispiel ein Doppelschneckenextruder, oder aber auch ein Prallreaktor vorgesehen werden, mittels denen ein effektiver Aufschluss und eine effektive Zerkleinerung der Biomasse möglich wird.

Gemäß einer besonders bevorzugten konkreten Ausgestaltung ist die zu vergärende stickstoffhaltige Biomasse Hühnertrockenkot. In diesem Zusammenhang ist es besonders vorteilhaft, wenn der im Hühnertrockenkot regelmäßig vorhandene hohe Sandanteil vor der Zuführung zur Mischeinrichtung, insbesondere im Nachgang zu einer der Mischeinrichtung vorgeschalteten Aufschluss- und/oder Zerkleinerungseinrichtung, mittels einer ein- oder mehrstufigen Sandabscheidevorrichtung reduziert bzw. entfernt wird. Dies kann grundsätzlich auf unterschiedliche Art und Weise geschehen, wobei eine Verfahrensführung vorteilhaft ist, bei der neben Sand auch auskristallisierte Harnsäure, welche ebenfalls für hohe Stickstoffwerte verantwortlich ist, aus dem Hühnertrockenkot abgeschieden werden kann.

Optional kann der von der Sandabscheidevorrichtung kommende Hühnertrockenkot einer Luftabscheidereinrichtung zugeführt werden, um Luft vom gereinigten Hühnertrockenkot abzuscheiden. Dadurch wird sichergestellt, dass keine unerwünschten Sauerstoffmengen in die Fermentationsstufe eingeschleust werden. Besonders bevorzugt ist weiter eine Verfahrensführung, bei der die abgeschiedene Luft ebenfalls dem zuvor erwähnten Gaswäscher zugeführt wird.

Gemäß einer weiteren besonders bevorzugten Ausgestaltung kann die Mischeinrichtung als Vorgrube mit einem bodenseitigen Sandfang ausgebildet sein, wobei die im Sandfang anfallenden Feststoffe vorzugsweise mittels einer Abzugseinrichtung zu vorgegebenen Zeiten abgezogen werden. Eine derartige Abzugseinrichtung kann zum Beispiel durch eine Absaugpumpe gebildet sein. Ein derartiger Sandfang kann gegebenenfalls bereits ausreichend sein, um den Sandanteil von zum Beispiel Hühnertrockenkot als zu vergärender stickstoffhaltiger Biomasse in ausreichendem Maße zu entfernen. Gegebenenfalls kann eine derartige Vorgrubenausgestaltung mit Sandfang somit auch alternativ zu der zuvor erwähnten optionalen Sandabscheidevorrichtung eingesetzt werden.

Die Fermentationsstufe selbst kann grundsätzlich ein- oder mehrstufig ausgebildet sein. Besonders bevorzugt ist jedoch eine zweistufige Fermentationsstufe mit einem ersten Fermenter und einem dem ersten Fermenter nachgeschalteten zweiten Fermenter als Nachfermenter. In Verbindung mit mehreren Fermentern kann der Abscheider- und/oder Seperatoreinrichtung grundsätzlich Gärrest aus einem oder beiden Fermentern zugeführt werden. Gemäß einer weiteren besonders bevorzugten Ausgestaltung kann der wenigstens eine Fermenter oder wenigstens einer der vorhandenen Fermenter mit einem bodenseitigen Sandfang ausgebildet sein, wobei die im Sandfang anfallenden Feststoffe vorzugsweise mittels einer Abzugseinrichtung zu vorgegebenen Zeiten abgezogen werden. Eine derartige Abzugseinrichtung kann auch hier wiederum zum Beispiel durch eine Absaugpumpe gebildet sein. Gegebenenfalls kann eine derartige Fermenterausgestaltung mit Sandfang somit auch alternativ zu der zuvor erwähnten optionalen Sandabscheidevorrichtung eingesetzt werden.

Bezüglich der Vorrichtung wird die vorstehend genannte erfindungsgemäße Aufgabe mit den Merkmalen des Patentanspruchs 13 gelöst, wobei vorteilhafte Ausgestaltungen Gegenstand der darauf rückbezogenen Unteransprüche sind.

Gemäß Anspruch 10 wird eine Vorrichtung zur Vergärung von stickstoffreicher Biomasse, insbesondere von Hühnertrockenkot in einer Biogasanlage vorgeschlagen, die eine Mischeinrichtung aufweist, der die zu vergärende stickstoffhaltige Biomasse und ein Rezirkulat zur Erzeugung eines Biomasse/Rezirkulat-Gemisches zuführbar ist. Weiter ist wenigstens ein der Mischeinrichtung, vorzugsweise direkt und unmittelbar, das heißt ohne weitere Behandlung und/oder Aufbereitung nachgeschalteter Fermenter vorgesehen, der die eigentliche Fermentationsstufe ausbildet. Dem Fermenter ist, vorzugsweise unmittelbar und direkt, das heißt ohne weitere Behandlung und/oder Aufbereitung eine Abscheider- und/oder Separatoreinrichtung nachgeschaltet, dem Gärrest aus dem wenigstens einen oder aus wenigstens einem der Fermenter zuführbar ist und in dem der Gärrest in eine Gärrest-Feststoffphase und in eine Gärrest-Flüssigphase aufgeteilt wird. Der Abscheider- und/oder Separatoreinrichtung ist weiter vorzugsweise wiederum unmittelbar und direkt eine Stickstoff-Ausbringeinrichtung nachgeschaltet, in der der Stickstoffgehalt in der Gärrest-Flüssigphase reduziert bzw. gegebenenfalls sogar im Wesentlichen entfernt werden kann. Schließlich umfasst die erfindungsgemäße Vorrichtung eine Rückführeinrichtung, mittels der die stickstoffreduzierte Gärrest-Flüssigphase als Rezirkulat der Mischeinrichtung zuführbar ist. Die Rückführeinrichtung umfasst vorzugsweise eine Rückführleitung und/oder einen Zwischenspeicher oder Pufferbehälter für die stickstoffreduzierte Gärrest-Flüssigphase.

Die sich mit einer derartigen Vorrichtung ergebenden Vorteile wurden bereits in Verbindung mit der erfindungsgemäßen Verfahrensführung ausführlich gewürdigt, sodass diesbezüglich auf die zuvor gemachten Ausführungen verwiesen wird. Das Gleiche gilt in analoger Weise für die hier nicht mehr im Detail wiedergegebenen Gegenstände der auf den Anspruch 13 rückbezogenen Unteransprüche.

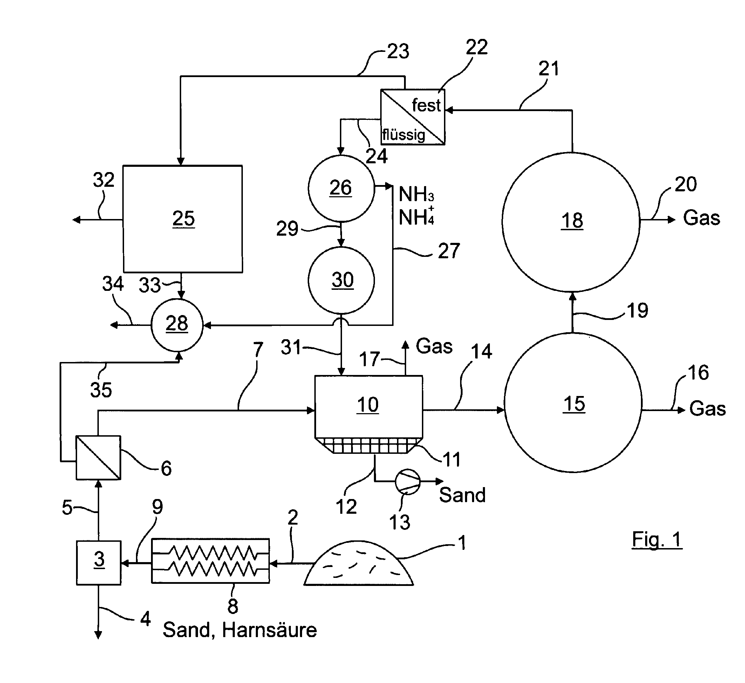

Die Erfindung wird nachfolgend anhand einer Zeichnung näher erläutert, in der die einzige Figur eine beispielhafte und schematische Ausgestaltung eines Ausführungsbeispiels der erfindungsgemäßen Verfahrensführung und der erfindungsgemäßen Vorrichtung zeigt.

Konkret zeigt die einzige Figur eine Bevorratung von Hühnertrockenkot 1, der neben einem hohen Sandanteil ferner auch auskristallisierte Harnsäure aufweist. Dieser Hühnertrockenkot 1 wird mittels einer geeigneten Transporteinrichtung 2, zum Beispiel einer Förderschnecke, einem Förderband oder dergleichen, einer Aufschluss- und/oder Zerkleinerungseinrichtung 8, zum Beispiel einem Extruder oder Prallreaktor zugeführt, indem der Hühnertrockenkot mechanisch zerkleinert sowie gegebenenfalls aufgeschlossen wird. Von dort aus wird der so vorbehandelte Hühnertrockenkot 9 dann über eine weitere geeignete Transporteinrichtung einem grundsätzlich beliebig ausbildbaren Sandabscheider 3 zugeführt, in dem unter Einsatz von Luft im Wesentlichen Sand sowie ggf. auch auskristallisierte Harnsäure abgeschieden wird, die entsprechend dem Pfeil 4, der jedwede geeignete Abzugseinrichtung symbolisiert, aus dem Sandabscheider 3 abgezogen wird bzw. werden. Der so gereinigte Hühnertrockenkot 5 kann, sofern dies erforderlich sein sollte, anschließend über eine geeignete Transporteinrichtung einem Luftabscheider 6 zugeführt werden, in dem eine definierte Menge eines aufgrund der Behandlung im Sandabscheider 3 im Hühnertrockenkot verbliebene Luftmenge 35 abgezogen wird. Die Luftabscheiderstufe ist hier, wie bereits zuvor ausgeführt, optional und hängt vom Anteil des verbliebenen Sauerstoffs im mittels des Sandabscheiders 3 vorbehandelten Hühnertrockenkot ab.

Mittels einer weiteren geeigneten Transporteinrichtung wird der so behandelte Hühnertrockenkot 7 anschließend einer als Vorgrube ausgebildeten Mischeinrichtung 10 zugeführt, in der der Hühnertrockenkot mit einem nachfolgend noch näher beschriebenen Rezirkulat 31 dergestalt vermischt wird, dass die Stickstoffkonzentration des so erzeugten Biomasse/Rezirkulat-Gemisches 14 unter einem definierten Stickstoff-Schwellwert liegt bzw. einen derartigen Stickstoff-Schwellwert nicht überschreitet.

Die als Vorgrube ausgebildete Mischeinrichtung 10 kann hier ferner mit einem bodenseitigen Sandfang 11 ausgebildet sein, sodass gegebenenfalls noch vorhandene Feststoffe, insbesondere Sand, im Sandfang 11 gesammelt werden. Dieser Sand kann dann über eine durch den Pfeil 12 symbolisierte Abzugseinrichtung, die vorzugsweise eine Absaugeinrichtung 13 aufweist, abgezogen werden. Im Falle des Vorsehens eines derartigen Sandfangs 11 kann gegebenenfalls auch auf den Einsatz des Fliehkraftabscheiders 3 und damit auf den Einsatz des Luftabscheiders 6 verzichtet werden, sodass dann der zu vergärende stickstoffreiche Hühnertrockenkot 1 zuerst direkt und unmittelbar der Aufschluss- und/oder Zerkleinerungseinrichtung 8 zugeführt wird.

In der Mischeinrichtung 10 wird ein pumpfähiges Biomasse/Rezirkulat-Gemisch 14 mit einem definierten Stickstoffanteil erzeugt, dass anschließend mittels einer Pumpeinrichtung (und einer entsprechenden Förderleitung) einem Fermenter 15 der Biogasanlage zugeführt wird, in dem die Biomasse vergärt und Biogas bzw. methanreiches Gas erzeugt wird, das mittels einer Abzugseinrichtung entsprechend Pfeil 16 vom Fermenter 15 abgezogen wird.

Gegebenenfalls in der Mischeinrichtung 10 gebildetes Biogas kann ebenfalls über eine Abzugseinrichtung entsprechend Pfeil 17 abgezogen werden, wie dies in der einzigen Figur schematisch dargestellt ist. Dem Fermenter 15 ist hier beispielhaft ein Nachfermenter 18 als Nachgärer nachgeschaltet, dem Gärrest 19 aus dem Fermenter 15 mittels einer geeigneten Transporteinrichtung 19 zugeführt wird. Im Nachfermenter 18 findet eine Nachvergärung der Biomasse statt, wobei das im Nachfermenter 18 entstehende Biogas (Pfeil 20) hier ebenfalls wiederum mittels einer Abzugseinrichtung abgezogen werden kann.

Aus dem Nachfermenter 18 wird, vorzugsweise entsprechend gesteuert bzw. geregelt mittels einer Steuer- und/oder Regeleinrichtung, die hier nicht dargestellt ist, zu vorgegebenen Zeiten und in vorgegebener Menge Gärrest 21 mittels einer geeigneten Transporteinrichtung einer Abscheider- und/oder Seperatoreinrichtung 22 zugeführt, in der der Gärrest in eine Gärrest-Feststoffphase 23 und in eine Gärrest-Flüssigphase 24 aufgeteilt bzw. separiert wird. Die Gärrest-Feststoffphase 23 wird zum einen mittels einer geeigneten Transporteinrichtung einer Trocknereinrichtung 25 zugeführt. Zum anderen wird die Gärrest-Flüssigphase 24 mittels einer geeigneten Transporteinrichtung einer zum Beispiel durch eine Strippereinrichtung ausgebildeten Stickstoff-Abzugseinrichtung 26 zugeführt, in der der in der Gärrest-Flüssigphase 24 gelöste bzw. gebundene Stickstoff, vorzugsweise in Form von Ammoniak (NH₃) und/oder in Form von Ammonium (NH₄⁺), in einem vorgegebenen Maße entfernt wird. Anschließend wird die so erhaltene Gasphase 27 mittels einer geeigneten Transporteinrichtung einem Gaswäscher 28 zugeführt, während die stickstoffreduzierte bzw. stickstoffarme Gärrest-Flüssigphase 29 nach der Stickstoff-Abzugseinrichtung 26 mittels einer geeigneten Transporteinrichtung einem Pufferbehälter 30 zugeführt wird.

Von dem Pufferbehälter 30 ausgehend wird, gesteuert bzw. geregelt mittels einer hier nicht dargestellten Steuer- und Regeleinrichtung, zu vorgegebenen Zeiten und in vorgegebener Menge stickstoffreduzierte Gärrest-Flüssigphase als Rezirkulat 31 abgezogen und mittels einer geeigneten Transporteinrichtung der Mischeinrichtung 10 zugeführt, in der das Biomasse/Rezirkulat-Gemisch 14 in der zuvor geschilderten Art und Weise mit dem gewünschten Stickstoffgehalt ohne den Einsatz von Frischwasser erzeugt wird.

Die in der Trocknereinrichtung 25 getrocknete Gärrest-Feststoffphase 32 kann einer weiteren Verwertung zugeführt werden, zum Beispiel als Dünger oder Brennstoff, während die Abluft 33 aus der Trocknereinrichtung 25 zusammen mit der Stickstoff-Gasphase 27 dem Gaswäscher 28 zugeführt wird, um dort, zum Beispiel unter Anwesenheit von Schwefelsäure (H₂SO₄) Ammoniumsulfat [(NH₄)₂SO₄] in fester oder flüssiger Form zu erzeugen, was in der einzigen Figur mit den Bezugszeichen 34 schematisch dargestellt ist. Der Gaswäscher 28 wird hier bevorzugt getrennt bezüglich der von der Trocknereinrichtung 25 kommenden Abluft 33 und der von der Stickstoff-Abzugseinrichtung 26 kommenden Gasphase 27 ausgeführt und/oder betrieben, so dass sich die dort ablaufenden jeweiligen Prozesse nicht wechselseitig behindern bzw. beeinflußen können. Grundsätzlich besteht aber auch die Möglichkeit, die Abluft 33 und die Gasphase zusammen in den Gaswäscher einzuführen und dort zu waschen.

Sofern ein Luftabscheider 6 vorhanden ist, kann die aus diesem abgezogene Abluft 35 ebenfalls mittels einer geeigneten Transporteinrichtung dem Gaswäscher 28 zugeführt werden.

Die zuvor gemachten Ausführungen zeigen, dass es mit der erfindungsgemäßen Verfahrensführung erstmalig gelingt, ohne Zufuhr von Frischwasser, stickstoffreiche Biomasse wie zum Beispiel Hühnertrockenkot, auf verfahrenstechnisch und apparatetechnisch einfache Weise zu vergären und damit auf effiziente und ökologisch wirksame Weise Biogas bzw. Methangas zu erzeugen.

### Bezugszeichenliste

- 1: Hühnertrockenkot
- 2: Transporteinrichtung
- 3: Sandabscheider
- 4: Pfeil
- 5: Transporteinrichtung
- 6: Luftabscheider
- 7: Transporteinrichtung
- 8: Ausschluss- und/oder Zerkleinerungseinrichtung
- 9: zerkleinerte Biomasse
- 10: Mischeinrichtung
- 11: Sandfang
- 12: Pfeil
- 13: Absaugeinrichtung
- 14: Biomasse/Rezirkulat-Gemisch
- 15: Fermenter
- 16: Abzugseinrichtung
- 17: Abzugseinrichtung
- 18: Nachfermenter
- 19: Gärrest
- 20: Abzugseinrichtung
- 21: Gärrest
- 22: Abscheider- und/oder Separatoreinrichtung
- 23: Gärrest-Festoffphase
- 24: Gärrest-Flüssigphase
- 25: Trocknereinrichtung
- 26: Stickstoff-Abzugseinrichtung
- 27: Stickstoff-Gasphase
- 28: Gaswäscher
- 29: stickstoffreduzierte Gärrest-Flüssigphase
- 30: Pufferbehälter
- 31: Rezirkulat
- 32: getrocknete Gärrest-Feststoffphase
- 33: Abluft
- 34: Ammoniumsulfat
- 35: Abluft

## Patentansprüche

1. Verfahren zur Vergärung von stickstoffhaltiger Biomasse, insbesondere von Hühnertrockenkot, in einer Biogasanlage, bei dem die zu vergärende stickstoffhaltige Biomasse (1) in einem ersten Schritt einer Mischeinrichtung (10) zugeführt wird, in der diese mit einer definierten Menge eines Rezirkulates (31) vermischt wird, insbesondere dergestalt, dass die Stickstoffkonzentration des so erzeugten Biomasse/Rezirkulat-Gemisches (14) unter einem definierten Stickstoff-Schwellwert liegt,
wobei das Biomasse/Rezirkulat-Gemisch (14) anschließend wenigstens einem Fermenter (15, 18) der Biogasanlage zugeführt wird, in dem Biogas erzeugt wird,
wobei in einem weiteren Verfahrensschritt Gärrest (21) aus dem wenigstens einen Fermenter (15, 18) oder aus wenigstens einem der Fermenter (18) abgezogen und einer Abscheider- und/oder Separatoreinrichtung (22) zugeführt wird, in der der Gärrest in eine Gärrest-Feststoffphase (23) und in eine Gärrest-Flüssigphase (24) separiert wird,
wobei lediglich die Gärrest-Flüssigphase (24) einer Stickstoff-Ausbringeinrichtung (26) zugeführt wird, in der der in der Gärrest-Flüssigphase (24) gelöste und/oder gebundene Stickstoff, vorzugsweise in Form von Ammonium und/oder Ammoniak, reduziert oder im Wesentlichen entfernt wird und eine stickstoffreduzierte, vorzugsweise eine im Wesentlichen stickstofffreie, Gärrest-Flüssigphase (29) erzeugt wird, und
dass die stickstoffreduzierte Gärrest-Flüssigphase (29) anschließend zu definierten Zeiten und in definierter Menge der Mischeinrichtung (10) als Rezirkulat (31) zugeführt wird.

2. Verfahren nach Anspruch 1, **dadurch gekennzeichnet, dass** die Stickstoff-Ausbringungseinrichtung (26) durch wenigstens eine Strippereinrichtung gebildet ist, in der der gelöste und/oder gebundene Stickstoff der Gärrest-Flüssigphase (24) in eine Gasphase (27) überführt und wenigstens eine stickstoffreduzierte, insbesondere im Wesentlichen stickstofffreie Gärrest-Flüssigphase (29) erzeugt wird.

3. Verfahren nach Anspruch 1, **dadurch gekennzeichnet, dass** die Stickstoff-Ausbringungseinrichtung durch wenigstens eine Umkehrosmoseeinrichtung gebildet ist, in der der gelöste und/oder gebundene Stickstoff der Gärrest-Flüssigphase durch Umkehrosmose abgetrennt und wenigstens eine strickstoffreduzierte, insbesondere im Wesentlichen stickstofffreie Gärrest-Flüssigphase erzeugt wird.

4. Verfahren nach einem der vorhergehenden Ansprüche, **dadurch gekennzeichnet, dass** die stickstoffreduzierte Gärrest-Flüssigphase (29) nach der Stickstoff-Ausbringungseinrichtung (26) in einem Zwischenspeicher oder Pufferbehälter (30) zwischengespeichert wird, aus dem zu definierten Zeiten eine definierte Menge der stickstoffreduzierten Gärrest-Flüssigphase (29) abgezogen und der Mischeinrichtung (10) als Rezirkulat (31) zugeführt wird.

5. Verfahren nach einem der vorhergehenden Ansprüchen, **dadurch gekennzeichnet, dass** die Gärrest-Feststoffphase (23) von der Abscheider- und/oder Separatoreinrichtung (22) abgezogen und einer Trocknereinrichtung (25) zugeführt wird, in der die Gärrest-Feststoffphase (23) auf einen definierten Trockensubstanzgehalt, insbesondere einen Trockensubstanzgehalt von 70 Prozent TS bis 95 Prozent TS, getrocknet wird.

6. Verfahren nach Anspruch 5, **dadurch gekennzeichnet, dass** die Abluft (33) aus der Trocknereinrichtung (25) zusammen mit dem aus der Gärrest-Flüssigphase entfernten Stickstoff (27), vorzugsweise in Form von Ammoniak und/oder Ammonium, wenigstens einem Gaswäscher (28) zugeführt wird, in dem, vorzugsweise unter Anwesenheit von Schwefelsäure, Ammoniumsulfat oder eine Ammoniumsulfatlösung hergestellt wird.

7. Verfahren nach einem der vorhergehenden Ansprüche, **dadurch gekennzeichnet, dass** die zu vergärende stickstoffhaltige Biomasse (1) vor deren Zuführung zur Mischeinrichtung (10) in einer Aufschluss- und/oder Zerkleinerungseinrichtung (8), insbesondere in einem Extruder und/oder einem Prallreaktor, aufgeschlossen und/oder zerkleinert wird und somit der Mischeinrichtung (10) als aufgeschlossene und/oder zerkleinerte stickstoffhaltige Biomasse zugeführt wird.

8. Verfahren nach einem der vorhergehenden Ansprüche, **dadurch gekennzeichnet, dass** die zu vergärende stickstoffhaltige Biomasse (1) Hühnertrockenkot ist, und dass der im Hühnertrockenkot vorhandene Sandanteil sowie ggf. darin enthaltene auskristallisierte Harnsäure vor der Zuführung zur Mischeinrichtung (10), vorzugsweise nach der Zuführung zu einer der Mischeinrichtung (10) vorgeschalteten Aufschluss- und/oder Zerkleinerungseinrichtung (8), mittels einer ein- oder mehrstufigen Sandabscheidevorrichtung (3) reduziert oder entfernt wird.

9. Verfahren nach einem der vorhergehenden Ansprüche, **dadurch gekennzeichnet, dass** die Mischeinrichtung (10) als Vorgrube mit einem bodenseitigen Sandfang (11) ausgebildet ist, wobei die im Sandfang (11) anfallenden Feststoffe mittels einer Abzugseinrichtung (13) zu vorgegebenen Zeiten abgezogen werden und/oder
dass einem ersten Fermenter (15) ein Nachfermenter (18) als zweiter Fermenter nachgeschaltet wird, wobei der Abscheider- und/oder Separatoreinrichtung (22) Gärrest (21) aus dem ersten und/oder zweiten Fermenter (15, 18) zugeführt wird.

10. Vorrichtung zur Vergärung von stickstoffreicher Biomasse, insbesondere von Hühnertrockenkot, in einer Biogasanlage, insbesondere zur Durchführung eines Verfahrens nach einem der vorhergehenden Ansprüche,
mit einer Mischeinrichtung (10), dem die zu vergärende stickstoffhaltige Biomasse (1) und ein Rezirkulat (31) zur Erzeugung eines Biomasse/Rezirkulat-Gemisches (14) zuführbar ist,
mit wenigstens einem der Mischeinrichtung (10) nachgeschalteten Fermenter (15, 18),
mit einer dem wenigstens einen Fermenter (15, 18) nachgeschalteten Abscheider- und/oder Separatoreinrichtung (22), dem Gärrest (21) aus dem wenigstens einen oder aus wenigstens einem der Fermenter (15, 18) zuführbar ist und in dem der Gärrest in eine Gärrest-Feststoffphase (23) und in eine Gärrest-Flüssigphase (24), aufgeteilt wird,
mit einer der Abscheider- und/oder Separatoreinrichtung (22) nachgeschalteten Stickstoff-Ausbringeinrichtung (26) zur Reduzierung oder Entfernung des gelösten und/oder gebundenen Stickstoffes, vorzugsweise in Form von Ammoniak und/oder Ammonium, aus der Gärrest-Flüssigphase (24), und
mit wenigstens einer, vorzugsweise eine Rückführleitung und/oder einen Zwischenspeicher oder Pufferbehälter (30) aufweisenden Rückführeinrichtung, mittels der die stickstoffreduzierte Gärrest-Flüssigphase (29) als Rezirkulat (31) der Mischeinrichtung (10) zuführbar ist.

11. Vorrichtung nach Anspruch 10, **dadurch gekennzeichnet, dass** die Stickstoff-Ausbringungseinrichtung (26) durch einen Strippereinrichtung oder eine Umkehrosmoseeinrichtung gebildet ist.

12. Vorrichtung nach Anspruch 10 oder 11, **dadurch gekennzeichnet, dass** der Mischeinrichtung (10) eine Aufschluss- und/oder Zerkleinerungseinrichtung (8) vorgeschaltet ist und/oder dass der Mischeinrichtung (10), vorzugsweise stromab einer der Mischeinrichtung (10) vorgeschalteten Aufschluss- und/oder Zerkleinerungseinrichtung (8), eine ein- oder mehrstufige Sandabscheidevorrichtung (3) vorgeschaltet ist.

13. Vorrichtung nach einem der Ansprüche 10 bis 12, **dadurch gekennzeichnet, dass** eine Trocknereinrichtung (25) vorgesehen ist, der die in der Abscheider- und/oder Separatoreinrichtung (22) abgeschiedene Gärrest-Feststoffphase (23) zuführbar ist.

14. Vorrichtung nach Anspruch 13, **dadurch gekennzeichnet, dass** wenigstens ein Gaswäscher (28) vorgesehen ist, dem Abluft aus der Trocknereinrichtung (25) und ein aus der Gärrest-Flüssigphase (24) mittels der Stickstoff-Ausbringeinrichtung (26) entfernter Stickstoff, vorzugsweise in Form von Ammoniak und/oder Ammonium, sowie gegebenenfalls weitere Abluft oder Gase aus dem Prozess zuführbar sind, insbesondere zur Herstellung von Ammoniumsulfat oder zur Herstellung einer Ammoniumsulfatlösung.

15. Vorrichtung nach einem der Ansprüche 10 bis 14, **dadurch gekennzeichnet, dass** die Fermentationsstufe mehrstufig durch einen ersten Fermenter (15) und wenigstens einen weiteren Fermenter (18) gebildet ist und/oder dass die Mischeinrichtung (10) als Vorgrube mit einem bodenseitigen Sandfang (11) ausgebildet ist, dem eine Sand-Abzugeinrichtung (13) zugeordnet ist.
